# EUROPEAN PATENT SPECIFICATION

(11) **EP 3 957 563 B1**
(45) Date of publication and mention of the grant of the patent: **11.02.2026**
(21) Application number: 21191483.3
(22) Date of filing: 16.08.2021
(51) Int. Cl.: B64D 11/00, B60N 2/00, A61B 5/00, A61B 5/02, A61B 5/024, B64D 11/06, B60N 2/64, B60N 2/75, A61B 5/01, A61B 5/1455, A61B 5/021, B60N 2/90

(54) **HEALTH MONITORING SEATING SYSTEMS**
SITZSYSTEME MIT GESUNDHEITSÜBERWACHUNG
SYSTÈMES DE SIÈGE POUR LA SURVEILLANCE DE LA SANTÉ

(30) Priority: 17.08.2020 IN 202041035388
(43) Date of publication of application: 23.02.2022
(73) Proprietor: Goodrich Aerospace Services Private Limited, 560048 Bangalore (IN); B/E Aerospace, Inc., Winston-Salem, NC 27105 (US)
(72) Inventor: KURUMPILAVU SUBRAMANIAN, Sanith, 560090 Chikkabanavara (IN); BATHLA, Dharamveer Surya Prakash, Sonepat (IN); MANDAVA, Raja, Bangalore (IN); SADASHIVAIAH, Nagesh, Kolar (IN); VISHWANNATHAN, Vishwanand, Bangalore (IN)
(74) Representative: Dehns

(56) References cited:
- WO-A1-2019/035337
- JP-A- 2017 029 219
- JP-B2- 3 766 302
- ESA SPACE SOLUTIONS: "Tempus - Medical Help Aboard Aircraft", 18 February 2014 (2014-02-18), XP055873546, Retrieved from the Internet <URL:https://www.youtube.com/watch?v=ZdbxOEr4QqY> [retrieved on 20211217]

## Description

### CROSS-REFERENCE TO RELATED APPLICATIONS

This application claims priority to U.S. Provisional Patent Application No. 63/029,009 filed May 22, 2020, and to U.S. Provisional Patent Application No. 63/030,484 filed May 27, 2020.

### BACKGROUND

### 1. Field

The present disclosure relates to aviation, aircraft interiors, and pandemic control, and more particularly to seating systems in aircraft for monitoring health of passengers.

### 2. Description of Related Art

The COVID-19 outbreak has increased the need to control the transit of infected individuals as well as the asymptomatic individuals carrying a viral charge. Due to the proximity of passengers to one another during air travel, and due to the duration of spending time together in that proximity, there is a particular need to make air travel as safe as possible from an epidemiological perspective.

The conventional techniques have been considered satisfactory for their intended purpose. However, there is an ever present need for improved systems and methods for monitoring the health of individuals during air travel. This disclosure provides a solution for this need. Esa Space Solutions, 18 February 20114 (2014-02-18), XP055873546, URL:https//www.youtube.com/watch?v=ZdbxOEr4Qqy describes medical help aboard an aircraft.

WO 2019/035337 A1 describes a biosensor.

JP2017029219 A describes a body temperature monitoring system for an in-flight passenger. In JP 2017029219 A, the temperature of an aircraft passenger seated on a seat is measured by a small-sized infrared camera attached to the back of a front seat. A feverish patient is detected from the measured temperature data, and displayed on a seat monitor installed in a flight attendant station together with a position of the seat so as to attract attention of flight attendants. A temperature history and an entry-exit history of the passenger, which are useful in determining whether or not the feverish patient needs to be isolated, are displayed on the seat monitor.

### SUMMARY

A system is provided as defined in claim 1. The system includes a passenger seat. A biometric sensor is configured to monitor at least one biometric of a passenger seated in the passenger seat. A controller is operatively connected to control the biometric sensor and to provide output indicative of a health condition of the passenger based on the biometric.

The passenger seat includes an armrest. The biometric sensor is mounted in the armrest. A second passenger seat is in front of the first passenger seat. The biometric sensor includes an imaging device mounted to the second passenger seat, facing back toward the first passenger seat, configured to monitor the first passenger seat for occupancy and temperature of occupant. The first passenger seat includes an armrest. A pulse oximeter is mounted in the armrest. The pulse oximeter is operatively connected to the controller to provide input relating to an occupant's pulse rate, heart rate, blood pressure, oxygen levels and/or vascular state. The controller is configured to use the input to provide output indicative of an occupant's health state. A display can be mounted in the headrest of the second passenger seat, facing toward the first passenger seat, and operatively connected to the controller to output information regarding the occupant's health state. The armrest can include a display operatively connected to the controller to output information regarding the occupant's health state. The first and second passenger seats are in a plurality of passenger seats, each having a respective pulse oximeter and a respective imaging device connected to a controller. A central display is operatively connected to the pulse oximeters and imaging devices to alert a crewmember of a health status of any occupant of the passenger seats.

A method is provided as defined by claim 8. The method is provided with a system according to claim 1 and includes checking a seat for occupancy. Upon detecting occupancy in the passenger seat, the method includes monitoring an occupant in the passenger seat for one or more biometrics indicative of a health condition.

Monitoring can include monitoring temperature of the occupant and outputting an alert if the temperature of the occupant is outside of a predetermined range. Monitoring can include monitoring images of the occupant and outputting an alert if the images are indicative of a respiratory tract infection. Monitoring can include monitoring pulse rate of the occupant and outputting an alert if the pulse rate is outside of a predetermined range. Monitoring can include monitoring blood oxygen level of the occupant and outputting an alert if the blood oxygen level is outside of a predetermined range. The method can include outputting an alert to take quarantine measures for the occupant if the one or more biometrics is/are indicative of a contagious condition.

These and other features of the systems and methods of the subject disclosure will become more readily apparent to those skilled in the art from the following detailed description of the preferred embodiments taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, preferred embodiments thereof will be described in detail herein below with reference to certain figures, wherein:
Fig. 1 is a schematic perspective view of an embodiment of a system constructed in accordance with the present disclosure, showing first and second passenger seats, with an occupant in the first passenger seat being monitored by a biometric sensor in the second passenger seat;
Fig. 2 is a schematic side elevation view of a biometric sensor for the system of Fig. 1, showing a pulse oximeter mounted in the armrest of one of the passenger seats;
Fig. 3 is a schematic view of a synoptic display for the system of Fig. 1; and
Fig. 4 is a flow diagram of the system of Fig. 1, showing a method of monitoring heath using the system of Fig. 1.

### DETAILED DESCRIPTION OF THE PREFERRED EMBODIMENTS

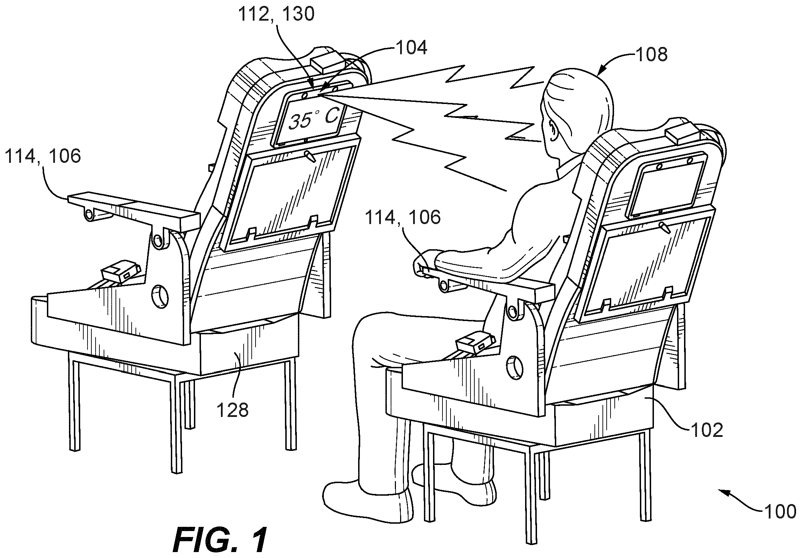

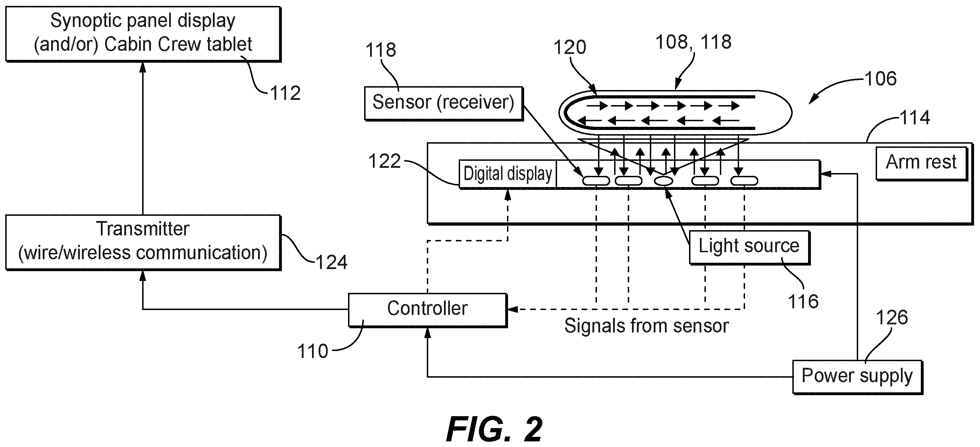

Reference will now be made to the drawings wherein like reference numerals identify similar structural features or aspects of the subject disclosure. For purposes of explanation and illustration, and not limitation, a partial view of an embodiment of a system in accordance with the claims is shown in Fig. 1 and is designated generally by reference character 100. Other embodiments of systems in accordance with the claims, are provided in Figs. 2-4, as will be described. The systems and methods described herein can be used to monitor health of passengers in transit.

The system 100 includes a first passenger seat 102. Biometric sensors 104, 106 are configured to monitor at least one biometric of a passenger 108 seated in the passenger seat 102. A controller 110 (labeled in Fig. 2) is operatively connected to control the biometric sensors 104, 106 and to provide output 112 indicative of a health condition of the passenger 108 based on the biometrics.

The passenger seat 102 includes an armrest 114. With reference to Fig. 2, the biometric sensor 106 includes a pulse oximeter mounted in the armrest 114. The pulse oximeter includes a light source 116 and receivers 118 is operatively connected to the controller 110 to provide input to the controller 110 relating to an seat occupant's pulse rate, heart rate, blood pressure, oxygen levels and/or vascular state, e.g. by scanning the finger 118 of the passenger 108 for blood flow 120. The controller 110 is configured to use the input to provide output 112 indicative of an occupant's health state. The armrest 114 includes a display 122 operatively connected to the controller 110 to output information regarding the occupant's health state. This information can also be output on a synoptic display, e.g. on an IFE as described below, or on a tablet for the flight crew, as also described below. A wired and/or wireless transmitter 124 can connect between the controller 110 and the devices displaying the output 112. A power supply 126 is connected to power the biometric sensor 106, controller 110, and transmitter 124.

With reference again to Fig. 1, a second passenger seat 128 is in front of the first passenger seat 102. Another biometric sensor 104 includes an imaging device mounted to the second passenger seat 128, facing back toward the first passenger seat 102, configured to monitor the first passenger seat 102 for occupancy and for temperature of the occupant or passenger 108. The imaging device can be configured to use visible or infrared image data to detect whether or not there is a passenger or occupant 108 in the first passenger seat 102. The imaging device can also include thermal imaging capability for use in thermal imaging the occupant 108 to detect temperature regularities indicative of respiratory tract infection, for example. An IFE (in-flight entertainment) display 130 is mounted in the headrest of the second passenger seat 128, facing toward the first passenger seat 102 for viewing by the occupant 108 seated in the first passenger seat 102. The display 130 is operatively connected to the controller 110 (shown in Fig. 2) to output information regarding the health state of the passenger or occupant 108. Those skilled in the art will readily appreciate that the first and second passenger seats 102, 128 can be just two in a plurality of similar passenger seats, each having a respective pulse oximeter 106 and a respective imaging device 104 connected to a controller 110.

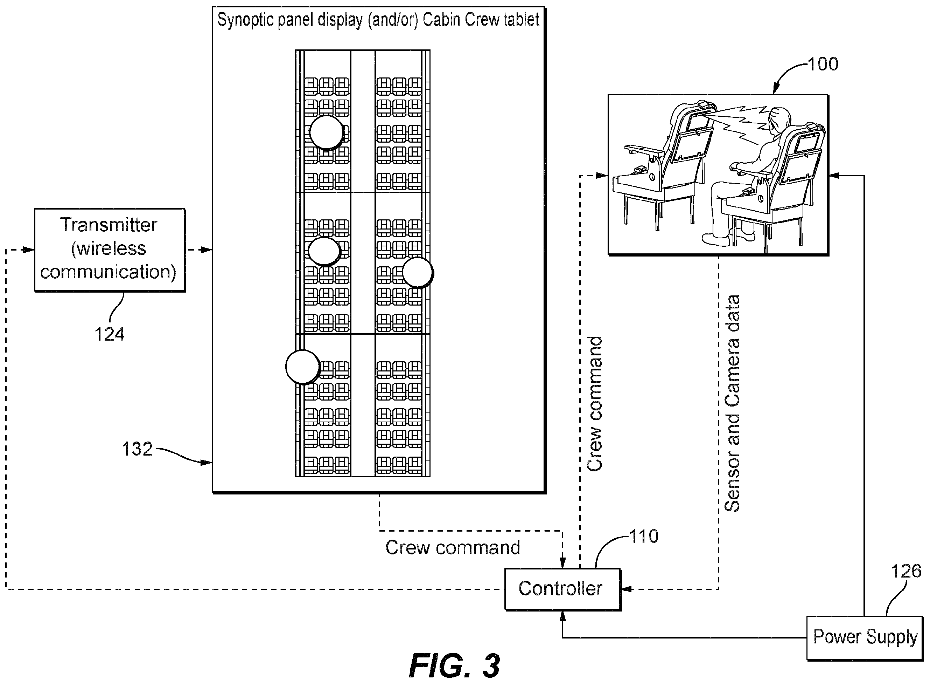

With reference now to Fig. 3, a central display, e.g., a tablet display 132 is operatively connected to the pulse oximeters 106 and imaging devices 104 (of Fig. 1) to alert a crewmember of a health status of any occupant of the passenger seats 102, 128. The view shown on the display 132 in Fig. 3 shows a floor plan of an aircraft cabin, highlighting the seats of passengers with potential health issues. The crew can issue commands from the tablet to the controller 110, e.g. to take quarantine measures such as altering air flow in the area of a cabin near passengers with potential contagions.

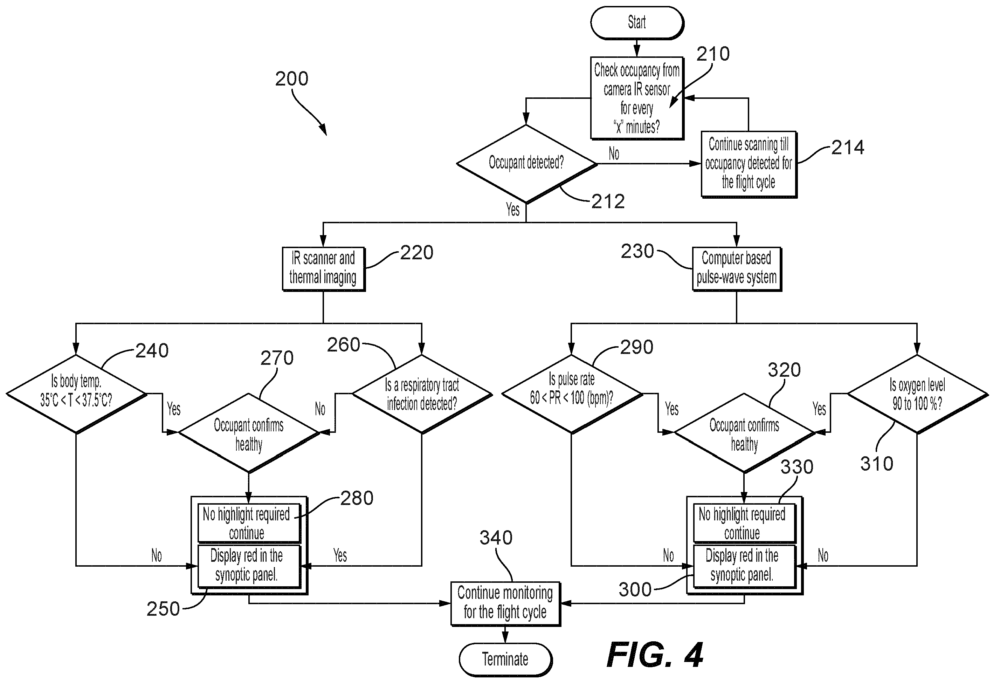

With reference now to Fig. 4, a method indicated by reference character 200 includes checking a seat for occupancy as indicated in box 210. This can be accomplished, e.g. using the IR capabilities of the sensor 104 described above. The check for seat occupancy can continue periodically in the event no passenger or occupant is detected, as indicated in Fig. 4 with boxes 212 and 214. Upon detecting occupancy in the passenger seat, the method includes monitoring an occupant in the passenger seat for one or more biometrics indicative of a health condition. This can include using the sensors 104, 106 described above, as indicated in Fig. 4 with the boxes 220 and 230.

Monitoring includes monitoring temperature of the occupant and outputting an alert if the temperature of the occupant is outside of a predetermined range, as indicated in Fig. 4 with boxes 240 and 250. For example, the passenger and/or crew can be alerted if the passenger's body temperature is reading outside the range 35°C to 37.7°C. Monitoring can also include monitoring images of the occupant and outputting an alert if the images are indicative of a respiratory tract infection, as indicated in boxes 260 and 250 in Fig. 4. If the body temperature and thermal imaging do not indicate a health issue, the system can output a confirmation that the passenger is healthy, as indicated in boxes 270 and 280 in Fig. 4.

Monitoring includes monitoring pulse rate of the occupant, e.g. using the sensor 106 shown in Fig. 2, and outputting an alert if the pulse rate is outside of a predetermined range, e.g. 60 BMP to 100 BPM as indicated in boxes 290 and 300 in Fig. 4. Monitoring can also include monitoring blood oxygen level of the occupant and outputting an alert if the blood oxygen level is outside of a predetermined range, e.g. below 90%, as indicated in boxes 310 and 300 in Fig. 4. If pulse rate and oxygen level are not indicative of a health issue, the system can output a confirmation that the passenger is healthy, as indicated in boxes 320 and 330 in Fig. 4. The monitoring can be repeated during a flight or other transit, as indicated in box 340. The method includes outputting an alert, e.g. to a crewmember using display 132 shown in Fig. 3. The crew member can take this as an indication to take quarantine or other suitable measures for the occupant if the one or more biometrics is/are indicative of a contagious condition.

The methods and systems of the present disclosure, as described above and shown in the drawings, provide for monitoring health of passengers in transit. While the apparatus and methods of the subject disclosure have been shown and described with reference to preferred embodiments, those skilled in the art will readily appreciate that changes and/or modifications may be made thereto without departing from the scope of the claims.

## Claims

1. A system comprising:
a plurality of seats comprising a first passenger seat (102) and a second passenger seat (128), wherein the second passenger seat (128) is in front of the first passenger seat (102), wherein the first passenger seat includes an armrest (114);
at least a biometric sensor (104,106) configured to monitor at least one biometric of an occupant (108) seated in the first passenger seat (102), wherein the biometric sensor (104,106) includes an imaging device (104) mounted to the second passenger seat (128), facing back toward the first passenger seat (102), configured to monitor the first passenger seat (102) for occupancy and temperature of the occupant, and wherein the biometric sensor (104,106) comprises an pulse oximeter (106) mounted in the armrest (114); and
a controller (110) operatively connected to the biometric sensors (104,106) to control the biometric sensors (104,106) and to provide output indicative of a health condition of the occupant based on the biometric;
wherein the pulse oximeter is operatively connected to the controller (110) to provide input relating to an occupant's pulse rate, heart rate, blood pressure, oxygen levels and/or vascular state, and wherein the controller (110) is configured to use the input to provide output indicative of an occupant's health state,
wherein said first passenger seat (102) and said second passenger seat (128) are in the plurality of passenger seats, each of the seats in the plurality of seats having a respective pulse oximeter (106) and a respective imaging device (104) connected to the controller (110), and
the system further comprising a central display (132) operatively connected to the pulse oximeters (106) and the imaging devices (104) to alert a crewmember of a health status of any occupant of the passenger seats (102,128).

2. The system as recited in claim 1, further comprising a display mounted in the headrest of the second passenger seat, facing toward the first passenger seat, and operatively connected to the controller to output information regarding the occupant's health state.

3. The system as recited in claim 1, wherein the armrest (114) includes a display operatively connected to the controller to output information regarding the occupant's health state.

4. A method (200) comprising:
providing a system (100) according to claim 1;
checking (210), by the imaging device (104), the first passenger seat for occupancy; and upon detecting occupancy in each of the first passenger seat, monitoring, by the biometric sensor (106), an occupant in the first passenger seat (102) for one or more biometrics indicative of a health condition.

5. The method as recited in claim 4, wherein monitoring by the biometric sensor (106) includes monitoring temperature of the occupants and outputting an alert if the temperature of the occupants is outside of a predetermined range.

6. The method as recited in claim 4, wherein monitoring by the imaging device (104) includes monitoring images of the occupants and outputting an alert if the images are indicative of a respiratory tract infection.

7. The method as recited in claim 4, wherein monitoring by the pulse oximeter (106) includes monitoring pulse rate of the occupants and outputting an alert if the pulse rate is outside of a predetermined range.

8. The method as recited in claim 4, wherein monitoring by the biometric sensor (106) includes monitoring blood oxygen level of the occupants and outputting an alert if the blood oxygen level is outside of a predetermined range.

9. The method as recited in claim 4, further comprising outputting by the central display (312) an alert to take quarantine measures for the occupant if the one or more biometrics is/are indicative of a contagious condition.

## Patentansprüche

1. System, umfassend:
eine Vielzahl von Sitzen, die einen ersten Passagiersitz (102) und einen zweiten Passagiersitz (128) umfassen, wobei sich der zweite Passagiersitz (128) vor dem ersten Passagiersitz (102) befindet, wobei der erste Passagiersitz eine Armlehne (114) beinhaltet;
mindestens einen biometrischen Sensor (104, 106), der konfiguriert ist, um mindestens ein biometrisches Merkmal eines in dem ersten Passagiersitz (102) sitzenden Insassen (108) zu überwachen, wobei der biometrische Sensor (104, 106) eine Bildgebungsvorrichtung (104) beinhaltet, die an dem zweiten Passagiersitz (128) montiert ist, nach hinten zu dem ersten Passagiersitz (102) weist und konfiguriert ist, um den ersten Passagiersitz (102) auf Belegung und Temperatur des Insassen zu überwachen, und wobei der biometrische Sensor (104, 106) ein Pulsoximeter (106) umfasst, das in der Armlehne (114) montiert ist; und
eine Steuerung (110), die operativ mit den biometrischen Sensoren (104, 106) verbunden ist, um die biometrischen Sensoren (104, 106) zu steuern und eine Ausgabe bereitzustellen, die indikativ für einen Gesundheitszustand des Insassen basierend auf dem biometrischen Merkmal ist;
wobei das Pulsoximeter operativ mit der Steuerung (110) verbunden ist, um eine Eingabe bereitzustellen, die sich auf eine Pulsfrequenz, Herzfrequenz, einen Blutdruck, Sauerstoffwerte und/oder einen Gefäßzustand eines Insassen bezieht, und wobei die Steuerung (110) konfiguriert ist, die Eingabe zu verwenden, um eine Ausgabe bereitzustellen, die indikativ für einen Gesundheitszustand eines Insassen ist,
wobei der besagte erste Passagiersitz (102) und der besagte zweite Passagiersitz (128) in der Vielzahl von Passagiersitzen sind, wobei jeder der Sitze in der Vielzahl von Sitzen ein jeweiliges Pulsoximeter (106) und eine jeweilige Bildgebungsvorrichtung (104) aufweist, die mit der Steuerung (110) verbunden sind, und
wobei das System ferner eine zentrale Anzeige (132) umfasst, die operativ mit den Pulsoximetern (106) und den Bildgebungsvorrichtungen (104) verbunden ist, um ein Besatzungsmitglied über einen Gesundheitsstatus eines beliebigen Insassen der Passagiersitze (102, 128) zu alarmieren.

2. System nach Anspruch 1, ferner umfassend eine Anzeige, die in der Kopfstütze des zweiten Passagiersitzes montiert ist, zu dem ersten Passagiersitz weist und operativ mit der Steuerung verbunden ist, um Informationen bezüglich des Gesundheitszustands des Insassen auszugeben.

3. System nach Anspruch 1, wobei die Armlehne (114) eine Anzeige beinhaltet, die operativ mit der Steuerung verbunden ist, um Informationen bezüglich des Gesundheitszustands des Insassen auszugeben.

4. Verfahren (200), umfassend:
Bereitstellen eines Systems (100) nach Anspruch 1;
Prüfen (210), durch die Bildgebungsvorrichtung (104), des ersten Passagiersitzes auf Belegung; und bei Erkennen einer Belegung in jedem der ersten Passagiersitze, Überwachen, durch den biometrischen Sensor (106), eines Insassen in dem ersten Passagiersitz (102) auf ein oder mehrere biometrische Merkmale, die indikativ für einen Gesundheitszustand sind.

5. Verfahren nach Anspruch 4, wobei das Überwachen durch den biometrischen Sensor (106) das Überwachen der Temperatur der Insassen und das Ausgeben eines Alarms beinhaltet, wenn die Temperatur der Insassen außerhalb eines vorbestimmten Bereichs liegt.

6. Verfahren nach Anspruch 4, wobei das Überwachen durch die Bildgebungsvorrichtung (104) das Überwachen von Bildern der Insassen und das Ausgeben eines Alarms beinhaltet, wenn die Bilder indikativ für eine Atemwegsinfektion sind.

7. Verfahren nach Anspruch 4, wobei das Überwachen durch das Pulsoximeter (106) das Überwachen der Pulsfrequenz der Insassen und das Ausgeben eines Alarms beinhaltet, wenn die Pulsfrequenz außerhalb eines vorbestimmten Bereichs liegt.

8. Verfahren nach Anspruch 4, wobei das Überwachen durch den biometrischen Sensor (106) das Überwachen des Blutsauerstoffgehalts der Insassen und das Ausgeben eines Alarms beinhaltet, wenn der Blutsauerstoffgehalt außerhalb eines vorbestimmten Bereichs liegt.

9. Verfahren nach Anspruch 4, ferner umfassend das Ausgeben, durch die zentrale Anzeige (312), eines Alarms, Quarantänemaßnahmen für den Insassen zu ergreifen, wenn das eine oder die mehreren biometrischen Merkmale indikativ für einen ansteckenden Zustand ist/sind.

## Revendications

1. Système comprenant :
une pluralité de sièges comprenant un premier siège passager (102) et un second siège passager (128), dans lequel le second siège passager (128) est situé devant le premier siège passager (102), dans lequel le premier siège passager inclut un accoudoir (114) ;
au moins un capteur biométrique (104, 106) configuré pour surveiller au moins une donnée biométrique d'un occupant (108) assis sur le premier siège passager (102), dans lequel le capteur biométrique (104, 106) inclut un dispositif d'imagerie (104) monté sur le second siège passager (128), orienté vers le premier siège passager (102), configuré pour surveiller l'occupation et la température de l'occupant du premier siège passager (102), et dans lequel le capteur biométrique (104, 106) comprend un oxymètre de pouls (106) monté dans l'accoudoir (114) ; et
un contrôleur (110) relié de manière opérationnelle aux capteurs biométriques (104, 106) pour contrôler les capteurs biométriques (104, 106) et fournir une sortie indicative de l'état de santé de l'occupant sur la base des données biométriques ;
dans lequel l'oxymètre de pouls est relié de manière opérationnelle au contrôleur (110) pour fournir des données d'entrée relative au pouls, à la fréquence cardiaque, à la pression artérielle, au niveau d'oxygène et/ou à l'état vasculaire d'un occupant, et dans lequel le contrôleur (110) est configuré pour utiliser les données d'entrée afin de fournir une sortie indicative de l'état de santé de l'occupant,
dans lequel ledit premier siège passager (102) et ledit second siège passager (128) font partie de la pluralité de sièges passagers, chacun des sièges de la pluralité de sièges étant équipé d'un oxymètre de pouls respectif (106) et d'un dispositif d'imagerie respectif (104) reliés au contrôleur (110), et
le système comprend également un écran central (132) relié de manière opérationnelle aux oxymètres de pouls (106) et aux dispositifs d'imagerie (104) pour alerter un membre d'équipage de l'état de santé de tout occupant des sièges passagers (102, 128).

2. Système selon la revendication 1, comprenant également un écran monté dans l'appui-tête du second siège passager, orienté vers le premier siège passager, et relié de manière opérationnelle au contrôleur pour afficher des informations concernant l'état de santé de l'occupant.

3. Système selon la revendication 1, dans lequel l'accoudoir (114) inclut un écran relié de manière opérationnelle au contrôleur pour afficher des informations concernant l'état de santé de l'occupant.

4. Procédé (200) comprenant :
la fourniture d'un système (100) selon la revendication 1 ;
le contrôle (210), par le dispositif d'imagerie (104), de l'occupation du premier siège passager ; et lors de la détection de l'occupation de chacun des premiers sièges passagers, la surveillance, par le capteur biométrique (106), d'un occupant du premier siège passager (102) pour une ou plusieurs données biométriques indiquant un état de santé.

5. Procédé selon la revendication 4, dans lequel la surveillance par le capteur biométrique (106) inclut la surveillance de la température des occupants et le déclenchement d'une alerte si la température des occupants est en dehors d'une plage prédéterminée.

6. Procédé selon la revendication 4, dans lequel la surveillance par le dispositif d'imagerie (104) inclut la surveillance des images des occupants et le déclenchement d'une alerte si les images sont indicatives d'une infection des voies respiratoires.

7. Procédé selon la revendication 4, dans lequel la surveillance par l'oxymètre de pouls (106) inclut la surveillance du pouls des occupants et le déclenchement d'une alerte si le pouls est en dehors d'une plage prédéterminée.

8. Procédé selon la revendication 4, dans lequel la surveillance par le capteur biométrique (106) inclut la surveillance du taux d'oxygène dans le sang des occupants et le déclenchement d'une alerte si le taux d'oxygène dans le sang est en dehors d'une plage prédéterminée.

9. Procédé selon la revendication 4, comprenant également le déclenchement par l'écran central (312) d'une alerte pour prendre des mesures de quarantaine pour l'occupant si l'une ou plusieurs données biométriques sont indicatives d'un état contagieux.
